# EUROPÄISCHE PATENTSCHRIFT

(11) **EP 1 757 927 B1**
(45) Veröffentlichungstag und Bekanntmachung des Hinweises auf die Patenterteilung: **18.11.2009**
(21) Anmeldenummer: 06450088.7
(22) Anmeldetag: 26.06.2006
(51) Int. Cl.: G01N 27/62, G01N 33/28

(54) **Verfahren und Vorrichtung zur Bestimmung der bei Kühlschmiervorgängen in die Atmosphäre emittierten Anteile an Schmierstoffen**
Method and apparatus for measuring the presence of cooling lubricant material in the atmosphere.
Méthode et appareil pour la mesure des émissions de lubrifiants de refroidissement dans l'athmosphère

(30) Priorität: 22.08.2005 AT 13902005
(43) Veröffentlichungstag der Anmeldung: 28.02.2007
(73) Patentinhaber: Palas GmbH Partikel-und Lasermesstechnik, 76229 Karlsruhe (DE)
(72) Erfinder: Höflinger, Wilhelm, Prof. Dr., 1060 Wien (AT); Wlaschitz, Peter, Dipl.-Ing., 1060 Wien (AT)
(74) Vertreter: Hehenberger, Reinhard

(56) Entgegenhaltungen:
- DE-A1- 10 162 278
- GB-A- 2 350 195
- RAYNOR PETER C ET AL: "Evaporation of polydisperse multicomponent oil droplets" AMERICAN INDUSTRIAL HYGIENE ASSOCIATION JOURNAL, Bd. 57, Nr. 12, 1996, Seiten 1128-1136, XP008090237 ISSN: 0002-8894
- RONNEBERG A ET AL: "Mortality and incidence of cancer among oil exposed workers in a Norwegian cable manufacturing company. Part I. Exposure conditions 1920-79." BRITISH JOURNAL OF INDUSTRIAL MEDICINE SEP 1988, Bd. 45, Nr. 9, September 1988 (1988-09), Seiten 589-594, XP008090231 ISSN: 0007-1072
- PIACITELLI G M ET AL: "Metalworking fluid exposures in small machine shops: an overview." AIHAJ : A JOURNAL FOR THE SCIENCE OF OCCUPATIONAL AND ENVIRONMENTAL HEALTH AND SAFETY 2001 MAY-JUN, Bd. 62, Nr. 3, Mai 2001 (2001-05), Seiten 356-370, XP008090269 ISSN: 1529-8663

## Beschreibung

Die gegenständliche Erfindung betrifft ein Verfahren zur Bestimmung der bei Kühlschmiervorgängen, insbesondere in der metallverarbeitenden Industrie, unter Verwendung von Kühlschmierstoffen in die Atmosphäre emittierten Anteile an Kühlschmierstoffen, wobei Proben der mit tröpfchenförmigen und dampfförmigen Anteilen an Kühlschmierstoffen belasteten Atmosphäre einem Verdampfer zugeführt werden, in welchem die in diesen Proben enthaltenen tröpfchenförmigen Anteile an Kühlschmierstoffen verdampft werden, wobei in der Folge der aus den tröpfchenförmigen Anteilen an Kühlschmierstoffen entstandene Dampf zusammen mit den in den Proben enthaltenen dampfförmigen Anteilen an Kühlschmierstoffen einem Analysegerät, insbesondere einem Flammenionisationsdetektor, zugeführt werden, durch welches die Konzentrationen der in den Proben enthaltenen Kühlschmierstoffe ermittelt werden, sowie eine Vorrichtung zur Durchführung dieses Verfahrens.

Bei der Metallverarbeitung besteht das Erfordernis, Kühlschmierstoffe einzusetzen, durch welche die Kontaktflächen zur Verminderung der Reibung geschmiert werden, weiters die bei der Verarbeitung auftretende Wärme abgeführt wird und zudem Späne u.dgl. abgefördert werden. Als Kühlschmierstoffe werden Mineralöle, natürliche Öle oder synthetische Öle bzw. Wasser-Öl-Emulsionen mit Ölanteilen von 2% bis 10% verwendet. Aufgrund ihrer hohen Kühlleistung und aufgrund des Sachverhaltes, dass sie keine Brand- bzw. Explosionsgefahr bedingen, sind Kühlschmierstoff-Emulsionen von besonderem Vorteil.

Durch die Verwendung von Kühlschmierstoffen bei der Metallverarbeitung wird allerdings die Atmosphäre durch tröpfchenförmige und dampfförmige Anteile an Kühlschmierstoffen, welche in diese emittiert werden, belastet. Hinsichtlich dieser Belastungen bestehen für die Konzentration von tröpfchenförmigen und von dampfförmigen Anteilen an Kühlschmierstoffen auf die Ölanteile bezogene normierte Werte für die maximalen Arbeitsplatzkonzentrationen (MAK), welche nicht überschritten werden dürfen. Aufgrund dieses Sachverhaltes besteht das Erfordernis, die Konzentrationen von die Atmosphäre belastenden Emissionen bei unterschiedlichen Arten und Zusammensetzungen von Kühlschmierstoffen, bei unterschiedlichen Arbeitsvorgängen, bei unterschiedlichen Betriebsbedingungen u.dgl. zu bestimmen, einerseits um die Einhaltung der bestehenden Normen zu gewährleisten und andererseits um Optimierungen in Bezug auf möglichst geringe Belastungen der Atmosphäre bei der Verwendung von Kühlschmierstoffen zu erzielen.

Es ist bekannt, Emissionen von Kühlschmierstoffen dadurch zu bestimmen, dass aus Proben der durch Kühlschmierstoffe belasteten Atmosphäre die tröpfchenförmigen Anteile mittels Filter abfiltriert werden und die dampfförmigen Anteile mittels Adsorberharzen gebunden werden, wobei durch Auswiegen der Filter, Eluieren der Adsorberharze und Analysieren des Eluates die in der Abluft enthaltenen Anteile an Kühlschmierstoffen ermittelt werden. Abgesehen davon, dass es sich dabei um aufwendige und zeitraubende Verfahren handelt, können jedoch durch diese Verfahren die Größen der tröpfchenförmigen Anteile nicht bestimmt werden.
Es ist weiters bekannt, die Anzahl und die Größen der in der Atmosphäre enthaltenen tröpfchenförmigen Anteile an Kühlschmierstoffen mittels eines Streulicht-Partikelzählers zu bestimmen. Dieses Verfahren ist gegenüber der Filtration deshalb vorteilhaft, da es umgehende Ergebnisse ermöglicht. Allerdings ist dieses Verfahren deshalb nicht den Erfordernissen entsprechend, da es in mehrfacher Hinsicht unrichtige Ergebnisse erbringt. Soferne nämlich die Emissionen von nicht in Wasser emulgierten Kühlschmierstoffen bestimmt werden, sind zwar die Messergebnisse hinsichtlich der in die Atmosphäre emittierten tröpfchenförmigen Anteile zutreffend. Allerdings werden durch einen Streulicht-Partikelzähler nur die tröpfchenförmigen Anteile und nicht die dampfförmigen Anteile gemessen.
Soferne hingegen die Emissionen von in Wasser emulgierten Kühlschmierstoffen bestimmt werden sollen, was im Hinblick auf die besonderen Vorteile von Kühlschmierstoffemulsionen in der Regel der Fall ist, haftet diesem Verfahren nicht nur der Nachteil an, dass die dampfförmigen Anteile nicht erfasst werden, sondern haftet diesem Verfahren auch der weitere Nachteil an, dass nur die Anzahl und die Größen der tröpfchenförmigen Anteile erfasst werden, dass aber deshalb, da die tröpfchenförmigen Anteile unterschiedliche Gehalte an Kühlschmierstoffen enthalten, hierdurch keine Aussagen über die Mengen an Kühlschmierstoffen in Volumenseinheiten erhalten werden können.

Aus der Literaturstelle Raynor Peter C. et al ,Evaporation of Polydisperse Multicomponent Oil Droplets', American Industrial Hygiene Association Journal, Bd.57, Nr.12, 1996, Seiten 1128 bis 1136, ISSN: 0002-8894, sowie aus der GB-A-2350185 AEA Technology plc veröffentlicht am 22. November 2000 ist es weiters bekannt, tropfenförmige und dampfförmige Öl-Verunreinigungen in der Atmosphäre dadurch zu bestimmen, dass die Öl-Verunreinigungen einem Verdampfer und hierauf einem Analysegerät zugeführt werden, worauf deren Konzentration bestimmt wird.

Der gegenständlichen Erfindung liegt somit die Aufgabe zugrunde, ein Verfahren und eine Vorrichtung zu schaffen, durch welche nicht nur der in einer Volumenseinheit enthaltene gesamte Gehalt an Kühlschmierstoffen, welcher in den Proben in Form von tröpfchenförmigen und dampfförmigen Anteilen enthalten ist, gemessen werden kann, wobei zudem das Messergebnis umgehend zur Verfügung steht, sondern auch die Anteile von Öltröpfchen mit unterschiedlichen Größen ermittelt werden können.

Diese Aufgaben werden erfindungsgemäß dadurch gelöst, dass die in den Proben enthaltenen tröpfchenförmigen Anteile an Kühlschmierstoffen nach vorgegebenen Größen fraktioniert und die nach ihren Größen fraktionierten tröpfchenförmigen Anteile jeweils gesondert dem Verdampfer zugeführt werden.
Vorzugsweise wird im Analysegerät die Anzahl der in Volumseinheiten der Proben enthaltenen Kohlenwasserstoffatome ermittelt. Vorzugsweise werden hierfür die Proben im Verdampfer auf 300°C bis 400°C erhitzt, wodurch die in diesen enthaltenen tröpfchenförmigen Anteile an Kühlschmierstoffen verdampft werden, worauf die Proben dem Analysegerät, insbesondere dem Flammenionisationsdetektor, mit einer Temperatur von 300°C zugeführt werden.

Durch dieses Verfahren können Proben von mit Kühlschmierstoffen belasteter Atmosphäre umgehend auf die Mengen der in diesen enthaltenen Anteile an Kühlschmierstoffen hin überprüft werden, wobei die Messergebnisse davon unabhängig sind, ob es sich um tröpfchenförmige oder dampfförmige Anteile an Kühlschmierstoffen handelt, wobei es unmaßgeblich ist, ob nicht wassergemischte Kühlschmierstoffe, sondern vielmehr Kühlschmierstoff-Emulsionen betroffen sind, und wobei weiters auch die tröpfchenförmigen Anteile ihrer Größe nach bestimmt werden. Dabei kann die Fraktionierung der Anteile nach Größen mittels sogenannten Impaktoren erfolgen.

Nach diesem Verfahren können aus den Proben der mit Kühlschmierstoffen belasteten Atmosphäre in aufeinanderfolgenden Schritten jeweils diejenigen tröpfchenförmigen Anteile, welche eine jeweils vorgegebenen Größe überschreiten, ausgesondert werden, wodurch in einem ersten Schritt nur diejenigen tröpfchenförmigen Anteile, welche eine bestimmte Größe nicht überschreiten sowie die in der betreffenden Probe enthaltenen dampfförmigen Anteile an Kühlschmierstoffen, erfasst werden. In weiteren Schritten werden jeweils die tröpfchenförmigen Anteile bis zu den jeweiligen maximalen Größen und die dampfförmigen Anteile erfasst. Durch Subtraktion der bei den jeweils vorhergehenden Schritten erbrachten Messwerte, können die in den betreffenden Proben befindlichen Gehalte an tröpfchenförmigen Anteilen von Kühlschmierstoffen in bestimmten Größenbereichen erfasst werden.
In einem letzten Schritt werden dem Verdampfer und dem Analysegerät eine Probe zugeführt, welche sämtliche tröpfchenförmigen und auch die dampfförmigen Anteile der in dieser Probe befindlichen Kühlschmierstoffe enthält. Durch Subtraktion der bei den jeweils vorhergehenden Schritten ermittelten Messwerte können die Mengen der tröpfchenförmigen Anteile in den betreffenden Größenbereichen ermittelt werden. Um die dampfförmigen Anteile an Kühlschmierstoffen in den Proben bestmöglich zu erfassen, wird die unterste Größe der tröpfchenförmigen Anteile möglichst klein gewählt, wobei sie z.B. im Bereich von 0,1 µm bis 0,5 µm liegt.

Vorzugsweise werden Proben einerseits dem Verdampfer und andererseits einem Streulicht-Partikelzähler zugeführt, wodurch auch die Größenverteilung der tröpfchenförmigen Anteile ermittelt werden kann.

Eine erfindungsgemäße Vorrichtung zur Durchführung dieses Verfahrens enthält einen Verdampfer und ein Analysegerät, insbesondere einen Flammenionisationsdetektor, welchen Proben der von Kühlschmierstoffen belasteten Atmosphäre zuführbar sind, wobei dem Verdampfer mehrere Einrichtungen zur Fraktionierung der Proben nach vorgegebenen Größen der tröpfchenförmigen Anteile an Kühlschmierstoffen vorgeschaltet sind und mittels einer Steuereinrichtung dem Verdampfer jeweils nur eine bestimmte Probe zuführbar ist. Vorzugsweise befindet sich in der Leitung zum Verdampfer ein der Förderung der Proben dienendes Gebläse, ist zusätzlich ein Streulicht-Partikelzähler vorgesehen und ist vorzugsweise der Verdampfer mit dem Analysegerät, insbesondere dem Flammenionisationsdetektor, mittels einer beheizten Leitung verbunden.

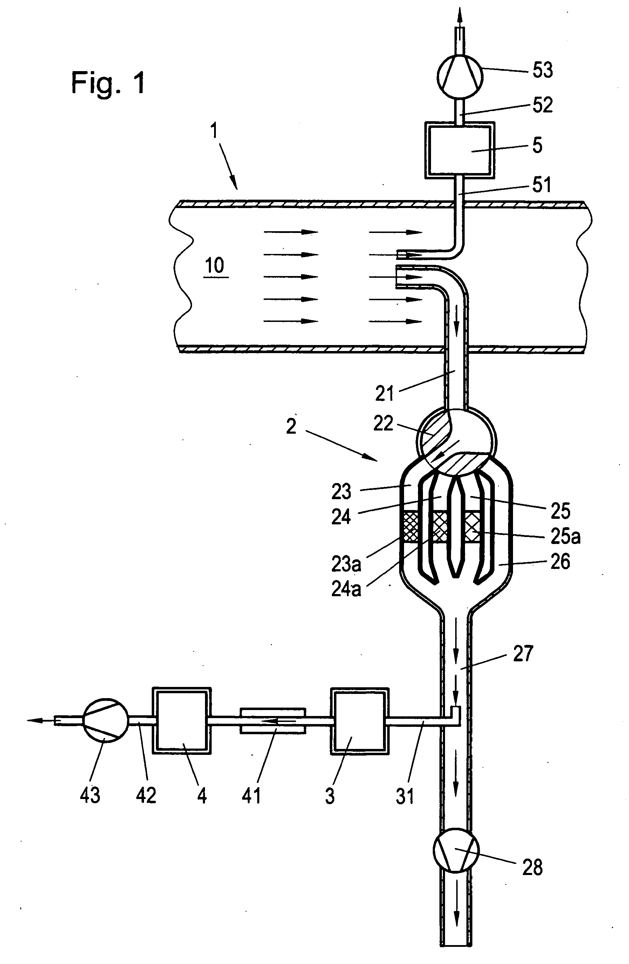

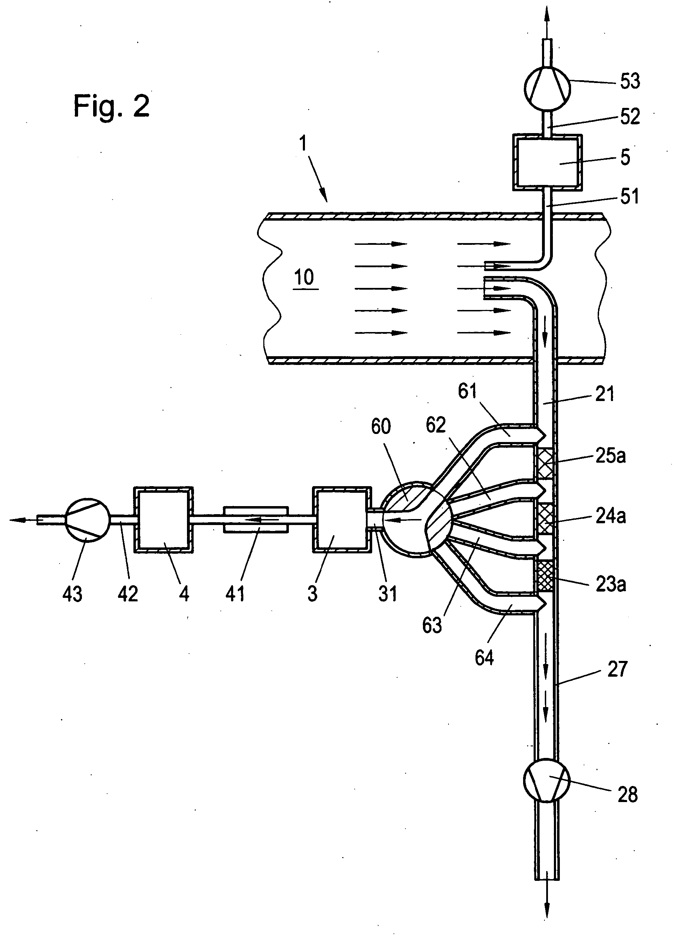

Das erfindungsgemäße Verfahren und eine erfindungsgemäße Vorrichtung sind nachstehend anhand von in den Fig.1 und Fig.2 der Zeichnung dargestellten Ausführungsbeispielen einer erfindungsgemäßen Vorrichtung näher erläutert.

Wie dies aus der Fig. 1 ersichtlich ist, ragt in den Innenraum 10 eines Kanals 1, in welchem mit tröpfchenförmigen und dampfförmigen Anteilen an Kühlschmierstoffen belastete Abluft strömt, eine zu einer Einrichtung 2 zur Fraktionierung der tröpfchenförmigen Anteile an Kühlschmierstoffen nach ihren Größen führende Leitung 21 ein. Die Einrichtung 2 enthält eine Mehrzahl von zueinander parallel verlaufenden Kanälen 23, 24, 25 und 26, durch welche über die Leitung 21 mittels einer der Einrichtung 2 vorgeschalteten Steuereinrichtung 22 wahlweise Proben der mit tröpfchenförmigen Anteilen und dampfförmigen Anteilen an Kühlschmierstoffen belasteten Abluft eingeleitet werden. In den Kanälen 23, 24 und 25 befinden sich Einrichtungen 23a, 24a und 25a, durch welche die in den Proben enthaltenen tröpfchenförmigen Anteile an Kühlschmierstoffen hinsichtlich ihrer Größen franktioniert werden. So werden z.B. durch die Einrichtung 23a sämtliche tröpfchenförmigen Anteile ausgeschieden, deren Durchmesser den Wert von 0.3 µm überschreitet, werden durch die Einrichtung 24a sämtliche tröpfchenförmigen Anteile ausgeschieden, deren Durchmesser den Wert 1 µm überschreitet und werden durch die Einrichtung 25a sämtliche tröpfchenförmigen Anteile ausgeschieden, deren Durchmesser den Wert 2 µm überschreitet. Diese Einrichtungen werden z.B. durch sogenannte Impaktoren gebildet.
Durch den Kanal 26 gelangen demgegenüber sämtliche in der betreffenden Probe enthaltenen tröpfchenförmigen und dampfförmigen Anteile an Kühlschmierstoffen hindurch. Die Kanäle 23, 24, 25 und 26 münden in eine Leitung 27, in welcher die mit tröpfchenförmigen und dampfförmigen Anteilen an Kühlschmierstoffen belastete Abluft mittels eines Gebläses 28 abgeführt wird.

In den Innenraum der Leitung 27 ragt eine Leitung 31 ein, welche zu einem Verdampfer 3 führt. Vom Verdampfer 3 führt eine beheizte Leitung 41 zu einem Analysegerät 4, welches z.B. durch einen Flammenionisationsdetektor gebildet ist. An das Analysegerät 4 schließt eine Leitung 42 an, in welcher sich ein Gebläse 43 befindet. In den Kanal 1 ragt weiters eine Leitung 51 ein, welche zu einem Streulicht-Partikelzähler 5 führt. Vom Streulicht-Partikelzähler 5 geht eine Leitung 52 ab, in welcher sich ein weiteres Gebläse 53 befindet.

### Die Wirkungsweise dieser Vorrichtung ist wie folgt:

Über die Leitung 21 werden mittels des Gebläses 28 aus dem Kanal 1, in welchen durch Kühlschmiervorgänge mit tröpfchenförmigen und dampfförmigen Anteilen an Kühlschmierstoffen belastete Abluft strömt, Proben dieser Abluft entnommen. Diese Proben werden mittels der Steuereinrichtung 22 durch einen der Kanäle 23, 24, 25 und 26 der Einrichtung 2 hindurchgeführt. Innerhalb der Kanäle 23, 24 und 25 befinden sich die Einrichtungen 23a, 24a und 25a zur Fraktionierung der in den Proben enthaltenen tröpfchenförmigen Anteile an Kühlschmierstoffen.

Durch die Einrichtung 23a werden z.B. sämtliche tröpfchenförmigen Anteile an Kühlschmierstoffen ausgeschieden, deren Durchmesser größer als 0,3 µm ist.
Durch die Einrichtung 24a werden z.B. sämtliche tröpfchenförmigen Anteile ausgeschieden, deren Durchmesser größer als 1 µm ist.

Durch die Einrichtung 25a werden z.B. sämtliche tröpfchenförmigen Anteile ausgeschieden, deren Durchmesser größer als 2 µm ist.
Durch den Kanal 26 gelangen demgegenüber sämtliche in den Proben enthaltenen tröpfchenförmigen und dampfförmigen Anteile an Kühlschmierstoffen hindurch.

In weiterer Folge werden Anteile dieser Proben über die Leitung 31 mittels des Gebläses 43 dem Verdampfer 3 zugeführt, in welchem die in diesen enthaltenen tröpfchenförmigen Anteile an Kühlschmierstoffen bei einer Temperatur von etwa 300°C bis 400°C verdampft werden. Der Ausgang des Verdampfers 3 ist über die vorzugsweise beheizte Leitung 41 zum Analysegerät 4 zur Bestimmung von in den Proben enthaltenen Mengen an Kohlenwasserstoff geführt.
Sofern eine in der Abluft enthaltene Probe in der Einrichtung 2 durch den Kanal 26 hindurchgeleitet wird, enthält sie sowohl die tröpfchenförmigen als auch die dampfförmigen Anteile an Kühlschmierstoffen. Durch das Analysegerät 4 werden sämtliche Kohlenwasserstoffatome erfasst, unabhängig davon, ob sie in der Probe in flüssiger oder in gasförmiger Phase enthalten waren. Hierdurch wird somit die Belastung der Atmosphäre durch die in diese aufgrund von Kühlschmiervorgängen gelangenden Anteile an Kühlschmierstoffen ermittelt.

Um die weitere Bestimmung dahingehend vorzunehmen, welche Größenverteilung die tröpfchenförmigen Anteile aufweisen und in welchen Ausmaßen einerseits die tröpfchenförmigen Anteile und andererseits die dampfförmigen Anteile an Kühlschmierstoffen in den Proben enthalten sind, werden aufeinanderfolgend aus der Abluft entnommene Proben durch die Kanäle 23, 24 und 25 hindurchgeleitet, wobei eine Fraktionierung der in den Proben enthaltenen tröpfchenförmigen Anteile nach ihren Größen erfolgt.

Da durch den Kanal 23 in den Verdampfer 3 nur diejenigen tröpfchenförmigen Anteile an Kühlschmierstoffen gelangen, deren Durchmesser kleiner als 0,3 µm ist, kann hierdurch in der betreffenden Probe der Gehalt an Kühlschmierstoffen, welche in denjenigen tröpfchenförmigen Anteilen, deren Durchmesser kleiner als 0,3 µm ist, und in den dampfförmigen Anteilen enthalten ist, ermittelt werden.
Da durch den Kanal 24 in den Verdampfer 3 nur diejenigen tröpfchenförmigen Anteile an Kühlschmierstoffen, deren Durchmesser geringer als 1 µm ist, sowie die dampfförmigen Anteile, durch den Kanal 25 in den Verdampfer 3 nur diejenigen tröpfchenförmigen Anteile an Kühlschmierstoffen, deren Durchmesser kleiner als 2 µm ist, sowie die jeweils dampfförmigen Anteile und durch den Kanal 26 sämtliche tröpfchenförmige und dampfförmige Anteile an Kühlschmierstoffen hindurch gelangen, können hierdurch entsprechende Ermittlungen vorgenommen werden.

Durch eine Subtraktion der Messergebnisse der jeweils vorangegangenen Proben können somit einerseits die in den Proben befindlichen dampfförmigen Anteile an Kühlschmierstoffen und andererseits die Gehalte der tröpfchenförmigen Anteile an Kühlschmierstoffen mit Durchmessern unter 0,3 µm, von 0,3 µm bis 1 µm, von 1 µm bis 2 µm und über 2 µm ermittelt werden. Weiters können hierdurch einerseits die dampfförmigen Anteile an Kühlschmierstoffen für sich und andererseits die gesamten tröpfchenförmigen Anteile an Kühlschmierstoffen für sich ermittelt werden.
Um die dampfförmigen Anteile an Kühlschmierstoffen erfassen zu können, wird der unterste Grenzwert möglichst klein gewählt. So kann sich z.B. der unterste Grenzwert im Bereich von 0,1 µm bis 0,5 µm befinden.

Um die Größenverteilungen der tröpfchenförmigen Anteile an Kühlschmierstoffen in den Proben zu ermitteln, werden über die Leitung 51 mittels des Ventilators 53 Anteile der Proben dem Streulicht-Partikelzähler 5 zugeführt. Hierdurch werden die Größenverteilungen der tröpfchenförmige Anteile an Kühlschmierstoffen in den einzelnen Proben ermittelt und werden diese mit den durch das Analysegerät 4 erbrachten Messergebnissen in Relation gesetzt.

In Fig.2 ist eine Ausführungsvariante der erfindungsgemäßen Vorrichtung dargestellt. Dabei befinden sich in der Leitung 21 aufeinanderfolgend der Impaktor 25a, durch welchen diejenigen tröpfchenförmigen Anteile, deren Durchmesser größer als 2 µm sind, ausgeschieden werden, weiters der Impaktor 24a, durch welchen diejenigen tröpfchenförmigen Anteile, deren Durchmesser größer als 1 µm sind, ausgeschieden werden und der Impaktor 23a, durch welchen diejenigen tröpfchenförmigen Anteile, deren Durchmesser größer als 0.3 µm sind, ausgeschieden werden.
Von der Leitung 21 zweigen Leitungen 61, 62 63 und 64 ab, welche zu einer Steuereinrichtung 60 führen, durch welche die durch die Leitungen 61 bis 64 gelangenden Proben dem Verdampfer 3 und dem Analysegerät 4 zugeführt werden, wodurch die Auswertungen erfolgen.
Die Verfahrensweise ist dabei diejenige, wie sie anhand der Fig. 1 beschrieben ist.

## Patentansprüche

1. Verfahren zur Bestimmung der bei Kühlschmiervorgängen, insbesondere in der metallverarbeitenden Industrie, unter Verwendung von Kühlschmierstoffen in die Atmosphäre emittierten Anteile an Kühlschmierstoffen, wobei Proben der mit tröpfchenförmigen und dampfförmigen Anteilen an Kühlschmierstoffen belasteten Atmosphäre einem Verdampfer (3) zugeführt werden, in welchem die in diesen Proben enthaltenen tröpfchenförmigen Anteile an Kühlschmierstoffen verdampft werden und wobei in der Folge der aus den tröpfchenförmigen Anteilen an Kühlschmierstoffen entstandene Dampf zusammen mit den in den Proben enthaltenen dampfförmigen Anteilen an Kühlschmierstoffen einem Analysegerät, insbesondere einem Flammenionisationsdetektor (4), zugeführt werden, durch welches die Konzentrationen der in den Proben enthaltenen Kühlschmierstoffe ermittelt werden, **dadurch gekennzeichnet, dass** die in den Proben enthaltenen tröpfchenförmigen Anteile an Kühlschmierstoffen nach vorgegebenen Größen fraktioniert und die nach ihren Größen fraktionierten tröpfchenförmigen Anteile jeweils gesondert dem Verdampfer (3) zugeführt werden.

2. Verfahren nach Patentanspruch 1, **dadurch gekennzeichnet, dass** im Analysegerät (4) die Anzahl der in Volumseinheiten der Proben enthaltenen Kohlenwasserstoffatome ermittelt wird.

3. Verfahren nach einem der Patentansprüche 1 und 2, **dadurch gekennzeichnet, dass** die Proben im Verdampfer auf 300°C bis 400°C erhitzt werden, wodurch die in diesen enthaltenen tröpfchenförmigen Anteile an Kühlschmierstoffen verdampft werden, worauf die Proben dem Analysegerät (4), insbesondere dem Flammenionisationsdetektor, mit einer Temperatur von etwa 300°C zugeführt werden.

4. Verfahren nach einem der Patentansprüche 1 bis 3, **dadurch gekennzeichnet, dass** der Gehalt an tröpfchenförmigen Anteilen in einem vorgegebenen Größenbereich dadurch ermittelt wird, dass vom Messergebnis die Ergebnisse der Messungen der vorangegangenen Bereiche mit geringeren Größen subtrahiert werden.

5. Verfahren nach einem der Patentansprüche 3 und 4, **dadurch gekennzeichnet, dass** der Gehalt an dampfförmigen Anteilen dadurch ermittelt wird, dass der unterste Wert bei der Fraktionierung der tröpfchenförmigen Anteile möglichst gering ist und z.B. im Bereich von 0,1 µm bis 0,5 µm liegt.

6. Verfahren nach einem der Patentansprüche 4 und 5, **dadurch gekennzeichnet, dass** die Fraktionierung der tröpfchenförmigen Anteile nach Größen mittels Impaktoren (23a, 24a, 25a) erfolgt.

7. Verfahren nach einem der Patentansprüche 1 bis 6, **dadurch gekennzeichnet, dass** Proben einerseits dem Verdampfer (3) und andererseits einem Streulicht-Partikelzähler (5) zugeführt werden.

8. Vorrichtung zur Durchführung des Verfahrens nach einem der Patentansprüche 1 bis 7, welche einen Verdampfer (3) und ein Analysegerät (4), insbesondere einen Flammenionisationsdetektor, enthält, welchen Proben der mit tröpfchenförmigen und dampfförmigen Anteilen an Kühlschmierstoffen belasteten Atmosphäre zuführbar sind, **dadurch gekennzeichnet, dass** dem Verdampfer (3) mehrere Einrichtungen (23a, 24a, 25a) zur Fraktionierung der Proben nach vorgegebenen Größen der tröpfchenförmigen Anteile an Kühlschmierstoffen vorgeschaltet sind und mittels einer Steuereinrichtung (22, 60) dem Verdampfer (3) jeweils nur eine bestimmte Probe zuführbar ist.

9. Vorrichtung nach Patentanspruch 8, **dadurch gekennzeichnet, dass** sich in der Leitung (21) ein der Förderung der Proben dienendes Gebläse (28) befindet.

10. Vorrichtung nach einem der Patentansprüche 8 und 9, **dadurch gekennzeichnet, dass** zusätzlich ein Streulicht-Partikelzähler (5) vorgesehen ist.

11. Vorrichtung nach einem der Patentansprüche 8 bis 10, **dadurch gekennzeichnet, dass** der Verdampfer (3) mit dem Analysegerät (4), insbesondere dem Flammenionisationsdetektor, über eine beheizte Leitung (41) verbunden ist.

## Claims

1. A method for determining the proportions of cooling lubricant released into the atmosphere when cooling lubricants are used in cooling and lubricating processes, in particular in the metal-processing industry, samples of the atmosphere contaminated with proportions of cooling lubricant in droplet and vapour form being fed to a vaporiser (3), in which the proportions of cooling lubricant in droplet form contained in these samples are vaporised, and the vapour arising from the proportions of cooling lubricant in droplet form being fed, together with the proportions of cooling lubricant in vapour form contained in the samples, to an analyser, in particular a flame ionisation detector (4), by means of which the concentrations of the cooling lubricants contained in the samples are determined, **characterised in that** the proportions of cooling lubricant in droplet form contained in the samples are fractionated according to predetermined sizes and the droplet-form proportions fractionated according to their sizes are in each case fed separately to the vaporiser (3).

2. A method according to claim 1, **characterised in that** the number of hydrocarbon atoms contained in unit volumes of the samples is determined in the analyser (4).

3. A method according to either one of claims 1 and 2, **characterised in that** the samples are heated to 300°C to 400°C in the vaporiser, whereby the proportions of cooling lubricant in droplet form contained in the samples are vaporised, whereupon the samples are fed to the analyser (4), in particular the flame ionisation detector, at a temperature of around 300°C.

4. A method according to any one of claims 1 to 3, **characterised in that** the content of proportions in droplet form in a given size range is determined **in that** the results of the measurements of the preceding ranges of smaller sizes are subtracted from the measurement result.

5. A method according to either one of claims 3 and 4, **characterised in that** the content of proportions in vapour form is determined **in that** the lowest value on fractionation of the proportions in droplet form is as small as possible, being for example in the range from 0.1 µm to 0.5 µm.

6. A method according to either one of claims 4 and 5, **characterised in that** fractionation of the proportions in droplet form according to size is performed by impactors (23a, 24a, 25a).

7. A method according to any one of claims 1 to 6, **characterised in that** samples are fed on the one hand to the vaporiser (3) and on the other hand to a scattered light particle counter (5).

8. A device for carrying out the method according to any one of claims 1 to 7, which comprises a vaporiser (3) and an analyser (4), in particular a flame ionisation detector, to which samples of the atmosphere contaminated with proportions of cooling lubricant in droplet and vapour form may be fed, **characterised in that** a plurality of means (23a, 24a, 25a) for fractionating the samples according to given sizes of the proportions of cooling lubricant in droplet form are connected upstream of the vaporiser (3) and in each case just one given sample may be fed to the vaporiser (3) by means of a controller (22, 60).

9. A device according to claim 8, **characterised in that** a blower (28) serving to convey the samples is located in the line (21).

10. A device according to either one of claims 8 and 9, **characterised in that** a scattered light particle counter (5) is additionally provided.

11. A device according to any one of claims 8 to 10, **characterised in that** the vaporiser (3) is connected to the analyser (4), in particular the flame ionisation detector, via a heated line (41).

## Revendications

1. Procédé pour définir les parts de lubrifiants de refroidissement qui sont émises dans l'atmosphère lors d'opérations de lubrification de refroidissement, en particulier dans l'industrie de transformation des métaux, à l'aide de lubrifiants de refroidissement, selon lequel des échantillons de l'atmosphère chargée de parts de lubrifiants de refroidissement sous forme de gouttelettes et de vapeur sont amenés dans un évaporateur (3), dans lequel les parts de lubrifiants sous forme de gouttelettes contenues dans ces échantillons sont évaporées, puis la vapeur formée à partir des parts de lubrifiants sous forme de gouttelettes est amenée avec les parts de lubrifiants sous forme de vapeur contenues dans les échantillons dans un appareil d'analyse, en particulier dans un détecteur d'ionisation de flammes (4), grâce auquel les concentrations de lubrifiants de refroidissement contenus dans les échantillons sont déterminées, **caractérisé en ce que** les parts de lubrifiants de refroidissement sous forme de gouttelettes contenues dans les échantillons sont fractionnées d'après des tailles prédéfinies, et les parts sous forme de gouttelettes fractionnées par tailles sont amenées séparément dans l'évaporateur (3).

2. Procédé selon la revendication 1, **caractérisé en ce que** dans l'appareil d'analyse (4), le nombre d'atomes d'hydrocarbure contenus dans les unités de volume des échantillons est déterminé.

3. Procédé selon l'une des revendications 1 et 2, **caractérisé en ce que** les échantillons sont chauffés dans l'évaporateur à 300°C-400°C, moyennant quoi les parts de lubrifiants de refroidissement sous forme de gouttelettes qui sont contenues dans ces échantillons sont évaporées, après quoi les échantillons sont amenés dans l'appareil d'analyse (4), en particulier dans le détecteur d'ionisation de flammes, à une température d'environ 300°C.

4. Procédé selon l'une des revendications 1 à 3, **caractérisé en ce que** la teneur en parts sous forme de gouttelettes dans une plage de tailles prédéfinie est déterminée grâce au fait que les résultats des mesures des plages précédentes de tailles plus faibles sont soustraits du résultat de mesure.

5. Procédé selon l'une des revendications 3 et 4, **caractérisé en ce que** la teneur en parts sous forme de vapeur est déterminée grâce au fait que la valeur minimale lors du fractionnement des parts sous forme de gouttelettes est aussi faible que possible et est située par exemple dans la plage de 0,1 µm à 0,5 µm.

6. Procédé selon l'une des revendications 4 et 5, **caractérisé en ce que** le fractionnement par tailles des parts sous forme de gouttelettes se fait à l'aide d'impacteurs (23a, 24a, 25a).

7. Procédé selon l'une des revendications 1 à 6, **caractérisé en ce que** des échantillons sont amenés d'une part dans l'évaporateur (3) et d'autre part dans un compteur de particules à diffusion de lumière (5).

8. Dispositif pour la mise en oeuvre du procédé selon l'une des revendications 1 à 7, qui contient un évaporateur (3) et un appareil d'analyse (4), en particulier un détecteur d'ionisation de flammes, dans lesquels peuvent être amenés des échantillons de l'atmosphère chargée de parts de lubrifiants de refroidissement sous forme de gouttelettes et de vapeur, **caractérisé en ce qu'**il est prévu, en amont de l'évaporateur (3), plusieurs dispositifs (23a, 24a, 25a) pour le fractionnement des échantillons d'après des tailles prédéfinies des parts de lubrifiants de refroidissement sous forme de gouttelettes, et seul un échantillon défini est apte à être amené à chaque fois dans l'évaporateur (3) à l'aide d'un dispositif de commande (22, 60).

9. Dispositif selon la revendication 8, **caractérisé en ce qu'**une soufflante (28) servant à l'acheminement des échantillons se trouve dans la conduite (21).

10. Dispositif selon l'une des revendications 8 et 9, **caractérisé en ce qu'**il est prévu en supplément un compteur de particules à diffusion de lumière (5).

11. Dispositif selon l'une des revendications 8 à 10, **caractérisé en ce que** l'évaporateur (3) est relié à l'appareil d'analyse (4), en particulier au détecteur d'ionisation de flammes, par une conduite chauffée (41).
